**Europäisches Patentamt**

**European Patent Office**

**Office européen des brevets**

(19)

(11) Publication number: **0 131 365**

**A1**

(12) **EUROPEAN PATENT APPLICATION**

(21) Application number: **84303591.6**

(22) Date of filing: **29.05.84**

(51) Int. Cl.⁴: **C 07 D 233/74**
**C 07 D 233/72**

(30) Priority: **31.05.83 JP 96407/83**

(43) Date of publication of application:
**16.01.85 Bulletin 85/3**

(84) Designated Contracting States:
**DE FR GB IT NL**

(71) Applicant: **AJINOMOTO CO., INC.**
**5-8, Kyobashi 1-chome, Chuo-ku**
**Tokyo 104(JP)**

(72) Inventor: **Yukawa, Toshihide**
**No. 50-40, Tana-cho Midori-ku**
**Yokohamashi Kanagawa-ken(JP)**

(72) Inventor: **Tsuji, Toshiaki**
**No. 5380, Oazahinaga**
**Yokkaichi-shi Mie-ken(JP)**

(72) Inventor: **Nagai, Takeshi**
**No. 5380, Oazahinaga**
**Yokkaichi-shi Mie-ken(JP)**

(74) Representative: **Bond, Bentley George et al,**
**HASELTINE LAKE & CO. Hazlitt House 28 Southampton**
**Buildings Chancery Lane**
**London, WC2A 1AT(GB)**

(54) A process for preparing a hydantoin derivative.

(57) An improved process is disclosed for preparing a hydantoin derivative from a hydrophobic aldehyde or ketone. In the process for preparing a hydantoin derivative by reaction of a hydrophobic aldehyde or ketone with ammonium, cyanide and carbonate ions in water, the reaction is carried out with said hydrophobic aldehyde or ketone emulsified in the presence of a surface-active agent or by vigorous mechanical agitation, whereby the corresponding hydantoin derivative is produced in excellent yields.

EP 0 131 365 A1

# A PROCESS FOR PREPARING A HYDANTOIN DERIVATIVE

The present invention relates to an improved process for preparing hydantoin derivative from a hydrophobic aldehyde or ketone.

The hydantoin derivative may be readily chemically hydrolyzed into DL-amino acid, followed, as required by optical resolution to afford the desired L- or D-isomer. Also, the hydantoin derivative can be enzymatically converted into optically α-amino acid, as disclosed in Japanese Patent Publication No. 13850/1962 and U.S. Patent No. 4,211,840.

The reaction to synthesize a hydantoin from the corresponding aldehyde or ketone (the Bucherer's hydantoin synthesis) is well known, in which the aldehyde or ketone is allowed to react with ammonium, cyanide and carbonate ions in an aqueous medium. When the aldehyde or ketone used as the starting material is hydrophobic, it is common practice to use a water-soluble organic solvent, such as alcohols, in order to permit the reaction to proceed in a homogeneous system (for example, Japanese Patent Publication No. 5702/1975), as otherwise polymerization and other side reactions would take place, particularly with aldehydes which are unstable under alkaline conditions, resulting in a drastically lowered product yield. The use of an organic solvent, however, necessitates its recovery step, thus complicating the entire process and adding to the cost for energy required for its recovery and other production costs.

We have found that it is possible to retard the aforementioned side reactions and therefore to avoid the reduction in yield of the intended reaction, if the contact of the hydrophobic material with the aqueous solution is effectively facilitated by the addition of a surface-active agent or by vigorous mechanical agitation, that is, if the reaction is carried out in an emulsified condition. This invention was based on these findings. Hydrophobic aldehydes or ketones defined in the present invention may be those having a solubility less than 10% by weight in water at 25°C. Examples of such aldehydes and ketones includes phenylacetaldehyde, benzaldehyde, acetophenone and methylisobutylketone.

In carrying out the process of the present invention, the reaction conditions, such as reaction temperature and time, and the ratio of the reactants, may be the same as those commonly adopted for hydantoin synthesis, or may be changed appropriately as required, except that the reaction is conducted in the absence of organic solvent and in an emulsified condition by the addition of a surface-active agent or by vigorous mechanical agitation. It is preferred that at least one mole of free ammonia or ammonium hydroxide based on the aldehyde or ketone is present in the reaction system. The preferred sources of ammonium ions are ammonia and ammonium cyanide.

Any water-soluble and ionized cyanide may be employed as a source of cyanide ions. From a practical point of view, hydrogen cyanide, ammonium cyanide, and the alkali metal cyanides, more specifically sodium and potassium cyanide, are preferred. The amount of ammonium and cyanide ions should be each at least one mole-equivalent per mole of aldehyde or ketone.

The source of carbonate ions is equally noncritical, but the carbonates and bicarbonates of sodium, potassium, or ammonium are preferred. The source of carbonate ions should be employed in an equimolecular amount based on the aldehyde or ketone or in a slight excess. Using more than 1.1 moles carbonate ion per mole of aldehyde or ketone has no measurable effect on the yield.

In conducting the hydantoin-forming reaction in the presence of a surface-active agent, particularly when starting from an aldehyde, it is preferable to add a small amount of the surface-active agent to an aqueous ammonia solution containing ammonium cyanide and ammonium bicarbonate, followed by addition of the aldehyde, and to allow the reaction to proceed in the emulsified condition.

Any types of surface-active agents (anionic, cationic, nonionic and amphoteric) are effective for the process of this invention. Typical examples include sodium dodecylbenzenesulfonate, dodecylmethylammonium chloride, N-lauryl-β-alanine, and polyoxyethylene(10) nonylphenyl ether.

The suitable amount of surface-active agent to be added is from 5% to 0.001%, preferably from 0.1% to 0.01%

—4—

based on the weight of the aldehyde or ketone. Since the agent is very effective with a small amount, its quantity to be used should be reduced to as low a level as practicable for ease of after-treatment.

When the reaction is carried out with vigorous mechanical agitation, it is preferable to add the aldehyde or ketone to an aqueous ammonia solution containing ammonium cyanide and ammonium carbonate (or bicarbonate) being vigorously agitated by means of a homogenizer or the like to keep the reaction system in a well emulsified condition. The formed emulsion is held at elevated temperature, preferably 40°C to130°C in a closed vessel until the desired hydantoin is formed.

As may be apparent from examples and comparative examples given later, the yield is only about 50-60% when the reaction is conducted with ordinary stirring (power requirement for agitation: 0.1 to 0.5 $KW/m^3$) in the absence of surface-active agent, while the desired hydantoin derivative can be formed in about 80% or higher yield if vigorous agitation is adopted (power requirement: 25 $KW/m^3$). The power requirement for agitation in this process should generally be 1 $KW/m^3$ or more, preferably 5 $KW/m^3$ or more. This can be accomplished on an industrial scale by, for example, mixing the aldehyde or ketone with an aqueous solution containing ammonium, cyanide and carbonate ions by means of a line mixer and continuously feeding the resulting mixture to a reactor.

From the foregoing it will be understood that if a hydrophobic aldehyde or ketone is reacted in an emulsified condition by the addition of a surface-active agent or by vigorous mechanical agitation, the corresponding hydantoin can be obtained in high yield comparable with that attained in a homogeneous system using an organic solvent. Thus, the process of this invention provides great advantages of eliminating the step of solvent recovery, with the resultant simplification of the entire manufacturing process and saving of the cost for solvent recovery, while maintaining a high level of yield. The hydantoin thus formed may be isolated from the reaction mixture before being subjected to hydrolysis or may be hydrolyzed without being isolated. Hydrolysis of hydantoin may be performed under known conditions, which may be appropriately modified as required. Isolation of the amino acid from the hydrolyzate can also be effected easily by known methods.

This invention is further illustrated by the following examples, in which determination of hydantoins and amino acids was made by using high-performance liquid chromatography (model: Hitachi 635A; column packing: Hitachi Gel #3011-0).

### Example 1

To 114 g of an aqueous solution containing 4.5 g (0.102 mole) of ammonium cyanide, 8.2 g (0.104 mole) of ammonium bicarbonate and 7.1 g of ammonia was added 0.011 g of sodium dodecylbenzenesulfonate (an anionic surface-active agent). After adding 11.3 g (0.094 mole) of phenylacetaldehyde to this solution under ordinary stirring condition, the resulting emulsion was heated in an autoclave at 80°C for 2 hours.

A portion of the reaction solution was sampled and analyzed, after concentration adjustment, for 5-benzylhydantoin by high-performance. liquid chromatography. The yield based on phenylacetaldehyde was 83%.

### Comparative Example 1

Another reaction carried out in much the same way as Example 1, except that 52 g of methanol was used in place of 0.011 g of sodium dodecylbenzenesulfonate, gave 5-benzylhydantoin in 83.5% yield.

### Comparative Example 2

A third reaction carried out in much the same way as Example 1, except that no sodium dodecylbenzenesulfonate was used, gave 5-benzylhydantoin in 54% yield.

### Example 2

To 114 g of an aqueous solution containing 4.5 g (0.102 mole) of ammonium cyanide, 8.2 g (0.104 mole) of ammonium bicarbonate and 3 g of ammonia being vigorously agitated in a homogenizer at a speed of 20,000 rpm, was added 11.3 g (0.094 mole) of phenylacetaldehyde at room temperature, and the resultant emulsion was heated in an autoclave at 80°C for 2 hours.

A portion of the reaction solution was sampled,

—7—

diluted appropriately and analyzed for 5-benzylhydantoin
by high- performance liquid chromatography.  The yield based on
phenylacetaldehyde was 82%.

### Example 3

To a mixture of 12 g of water and a solution
consisting of 4.2 g (0.095 mole) of ammonium cyanide, 8.0 g
(0.101 mole) of ammonium bicarbonate and 58 g of 10 wt%
ammonia water, was added 0.009 g of sodium dodecylbenzenesulfonate (an anionic surface-active agent), then 8.5 g
(0.080 mole) of benzaldehyde was added to this solution
at room temperature with ordinary stirring, and the
resultant emulsion was heated in an autoclave at 120°C for
30 minutes.

A portion of the reaction solution was sampled,
diluted appropriately and analyzed for 5-phenylhydantoin
by high- performance liquid chromatography.  The yield based on
benzaldehyde was 87.5%.

### Comparative Example 3

Another reaction carried out in much the same
way as Example 3, except that 100 g of methanol was used
in place of 0.009 g of sodium dodecylbenzenesulfonate, gave
5-phenylhydantoin in 89% yield.

### Comparative Example 4

A third reaction carried out in much the same
way as Example 3, except that no sodium dodecylbenzenesulfonate was used, gave 5-phenylhydantoin in 50% yield.

-9-

## Example 4

To a mixture of 12 g of water and 74.2 g of a solution containing 4.2 g (0.095 mole) of ammonium cyanide, 8.0 g (0.101 mole) of ammonium bicarbonate and 58 g of 10 wt% ammonia water being vigorously agitated in a homogenizer at a speed of 20,000 rpm, was added 8.5 g (0.080 mole) of benzaldehyde at room temperature, and the resultant emulsion was heated in an autoclave at 120°C for 30 minutes.

A portion of the reaction solution was sampled, diluted appropriately, and analyzed for 5-phenylhydantoin by high-performance liquid chromatography. The yield based on benzaldehyde was 87%.

## Example 5

To a mixture of 12 g of water and 74.2 g of a solution containing 4.2 g (0.095 mole) of ammonium cyanide, 8.0 g (0.101 mole) of ammonium bicarbonate and 58 g of 20 wt% ammonia water, was added 0.009 g of sodium dodecylbenzenesulfonate (an anionic surface-active agent), then 8.0 g (0.080 mole) of methylisobutylketone was added to this solution at room temperature with ordinary stirring, and the resultant emulsion was heated in an autoclave at 70°C for 2 hours.

A portion of the reaction solution was sampled, diluted appropriately and analyzed for 5-methyl-5-isobutylketone by high-performance liquid chromatography. The yield based on methylisobutylketone was 80%.

## Comparative Example 5

Another reaction carried out in much the same way as Example 5, except that 100 g of methanol was used in place of 0.009 g of sodium dodecylbenzenesulfonate, gave 5-methyl-5-isobutylketone in 82% yield.

## Comparative Example 6

A third reaction carried out in much the same way as Example 5, except that no sodium dodecylbenzenesulfonate was used, gave 5-methyl-5-isobutylketone in 60% yield.

## Example 6

To 114g of an aqueous solution containing 4.5g ( 0.102 mole ) of ammonium cyanide, 8.2g ( 0.104 mole ) of ammonium bicarbonate and 7.1g of ammonia was added 0.011g of sodium dodecylbenzenesulfonate ( an anionic surface-active agent ). After adding 11.3g ( 0.094 mole ) of phenylacetaldehyde to this solution under ordinary stirring condition, the resulting emulsion was heated in an autoclave at 120°C for 30 minutes. An aqueous 3N-NaOH solution ( 109ml ) was then added and the mixture was heated at 160°C for an additional one and a half hours.

A portion of the reaction solution was sampled and analyzed, after concentration adjustment, for DL-phenyl-alanine by high-(performance)-liquid chromatography. The yield based on phenylacetaldehyde was 99.2%.

## Comparative Example 7

Another reaction carried out in much the same way as Example 6, except that 52g of methanol was used in place of 0.011g of sodium dodecylbenzenesulfonate, gave DL-phenylalanine in 99.5%.

## Comparative Example 8

A third reaction carried out in much the same way as Example 6, except that no sodium dodecylbenzenesulfonate was used, gave DL-phenylalanine in 70% yield.

## Example 7

To 114g of an aqueous solution containing 4.5g ( 0.102 mole ) of ammonium cyanide, 8.2g ( 0.104 mole ) of ammonium bicarbonate and 7.1g of ammonia being vigorously agitated in a homogenizer at a speed of 20,000rpm, was added 11.3g ( 0.094 mole ) of phenylacetaldehyde at room temperature, and the resultant emulsion was heated in an autoclave at 120°C for 30 minutes. After addition of 33g of 48% aqueous caustic soda solution and 250g of water, the mixture was heated at 160°C for two hours to effect hydrolysis.

A portion of the reaction solution was sampled, diluted appropriately and analyzed for DL-phenylalanine by high-performance liquid chromatography. The yield based on phenylacetaldehyde was 99.3%.


### Example 8

An aqueous solution ( 300g ) containing DL-phenylalanine, obtained according to the method of Exzample.6 , was
concentrated under reduced pressure to a volume of 100ml,
from which crystals separated out by adding 26.6g of 60%
sulfuric acid to reduce the pH to 6.8.

After collection by filtration and drying, 14.2g of
crystals were obtained, which were identified as DL-phenyl-
alanine by comparison of IR spectrum with an authentic
sample and by other methods.   The yield based on phenylacetaldehyde was 91.5%.


### Example 9

To a mixture of 12g of water and a solution consisting
of 4.2g ( 0.095 mole ) of ammonium cyanide, 8.0g ( 0.101
mole ) of ammonium bicarbonate and 58g of 20-wt% ammonia
water, was added 0.009g of sodium dodecylbenzenesulfonate
( an anionic surafce-active agent ), then 8.5g ( 0.030 mole
) of benzaldehyde was added to this solution at room temperature with ordinary stirring, and the resultant emulsion
was heated in an autoclave at 120°C for 30 minutes.   After
addition of 33g of 48% aqueous caustic soda solution and
250g of water, the mixture was further heated at 160°C for two hours to effect hydrolysis.

A portion of the reaction solution was sampled, diluted appropriately and analyzed for DL-phenylglycine by high-performance liquid chromatography.   The yield based on benzaldehyde was 95.7%.


### Comparative Example 9

A reaction carried out in much the same way as Example 9 , except that 100g of methanol was used in place of 0.009g of sodium dodecylbenzenesulfonate, gave DL-phenylglycine in 95.3% yield.


### Comparative Example 10

A third reaction carried out in much the same way as Example 9, except that no sodium dodecylbenzenesulfonate was used, gave DL-phenylglycine in 55% yield.


### Example 10

To a mixture of 12g of water and 74.2g of a solution containing 4.2g ( 0.095 mole ) of ammonium cyanide, 8.0g ( 0.101 mole ) of ammonium bicarbonate and 53g of 20-wt% ammonia water being vigorously agitated in a homogenizer at a speed of 20,000rpm, was added 8.5g ( 0.030 mole ) of benzaldehyde at room temperature, and the resultant emulsion was heated in an autoclave at 120°C for 30 minutes.   After addition of 33g of 43% aqueous caustic soda solution and 250g of water, the mixture was further heated at 160°C for two hours to complete hydrolysis.

A portion of the reaction solution was sampled, diluted appropriately, and analyzed for DL-phenylglycine by high-performance liquid chromatography. The yied based on benzaldehyde was 95.7%.

**0131365**

-14-

**CLAIMS**

1. A process for preparing a hydantoin derivative, which comprises reacting a hydrophobic aldehyde or ketone simultaneously with ammonium, cyanide and carbonate ions in water or an aqueous medium at elevated temperature, characterised in that the reaction is carried out with said hydrophobic aldehyde or ketone in an emulsified state due to the presence of a surface-active agent in the reaction mixture and/or due to vigorous mechanical agitation of the reaction mixture.

2. A process according to claim 1, wherein said hydrophobic aldehyde is phenylacetaldehyde or benzaldehyde.

3. A process according to claim 1, wherein said hydrophobic ketone is acetophenone or methylisobutylketone.

4. A process according to any of claims 1 to 3, wherein the amount of said surface-active agent is from 5% to 0.001% based on the weight of the aldehyde or ketone.

5. A process according to any of claims 1 to 4, wherein the power requirement for agitation is at least 1 KW/m$^3$.

6. A hydantoin derivative when prepared by a process according to any of claims 1 to 5.

**European Patent Office**

# EUROPEAN SEARCH REPORT

**0131365**
Application number

EP 84 30 3591

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-2 354 327 (CIBA-GEIGY) * page 6, example A * | 1 | C 07 D 233/74 C 07 D 233/72 |
| X | FR-A-1 164 184 (CASSELLA) * examples 1,2,3 * | 1 | |
| X | FR-A-2 345 430 (LAFON) * page 22, example 11 * | 1 | |
| A | GB-A- 644 800 (PARKE, DAVIS & CO.) | | |

|  | TECHNICAL FIELDS SEARCHED (Int. Cl. ³) |
|---|---|
|  | C 07 D 233/00 |

The present search report has been drawn up for all claims

| Place of search THE HAGUE | Date of completion of the search 30-08-1984 | Examiner DE BUYSER I.A.F. |
|---|---|---|